# EUROPEAN PATENT APPLICATION

(11) **EP 1 477 171 A1**
(43) Date of publication of application: **17.11.2004**
(21) Application number: 04011701.2
(22) Date of filing: 17.05.2004
(51) Int. Cl.: A61K 31/70, A61P 1/00, A23L 1/30

(54) **Intestinal mineral absorption capacity improver**

(30) Priority: 11.11.2003 JP 2003380653; 16.05.2003 JP 2003138986
(71) Applicant: Kao Corporation, Tokyo 103-8210 (JP)
(72) Inventor: Fujinaka, Hidetake C/o Kao Corporation, Haga-gun Tochigi 321-3497 (JP); Nakamura, Junji C/o Kao Corporation, Haga-gun Tochigi 321-3497 (JP); Murase, Daiki C/o Kao Corporation, Haga-gun Tochigi 321-3497 (JP); Souno, Hatsumi C/o Kao Corporation, Haga-gun Tochigi 321-3497 (JP); Kobayashi, Hisataka C/o Kao Corporation, Haga-gun Tochigi 321-3497 (JP)
(74) Representative: HOFFMANN - EITLE

(57) **Abstract**

The present invention relates to an intestinal mineral absorption capacity improver containing glucose 1-phosphate sodium salt as an effective ingredient.

## Description

### Field of the Invention

The present invention relates to drugs or foods for improving the function of the intestinal tract.

### Background of the Invention

Minerals such as potassium, calcium, magnesium, iron, copper, zinc, cobalt and manganese are vital nutrients for humans, but recent tendency to widespread consumption of processed foods has led to mineral deficiency. The mineral deficiency can be corrected by taking minerals and for this purpose, a number of mineral supplements are put on the market. Calcium hexose phosphate is known as such a mineral supplement (Japanese Patent Application Laid-Open No. Sho 64-22). Even if mineral supplements are administered, however, minerals are not absorbed in the body efficiently when the absorption capacity from the intestinal tract is impaired.

Enhancers for mineral absorption in the intestinal tract have therefore been developed. They have two types, that is, for enhancing absorption capacity by converting the form of minerals into more absorptive one; and for activating mineral absorption or transport mechanism in the intestinal tract. Examples of the former one include casein phosphor peptide, fructo-oligosaccharde, CCM and sugar phosphate ester (Japanese Patent Application Laid-Open No. Hei 2-249468). As the latter one, only an activated vitamin D formulation is known as a drug for Activating/enhancing the mineral transport system.

In the former type conventional mineral absorption enhancer which converts the form of minerals into a more absorptive one, the enhancer and mineral supplement must be administered simultaneously to convert the form of minerals into more absorptive one, because the effective ingredient of the enhancer must exist in the vicinity of the minerals in the intestinal tract after administration to ensure their reaction. When they exist in the intestinal tract, a temporary rise in the amount of absorbed minerals can be expected, but with the passage of time after meal, the effective ingredient disappears from the intestinal tract and the above-described effect cannot be expected at all. On the other hand, activated vitamin D is known as only one drug that is continuously effective for enhancing calcium absorption in the intestinal tract when administered continuously. It is however known to cause side effects such as weight loss due to hypercalcemia so that a sufficient care must be taken upon its use (Weber K., et al: J. Bone Miner. Res., 10(4), 639-651 (2001)).

### Summary of the Invention

In the present invention, there is thus provided an intestinal mineral absorption capacity improver having glucose 1-phosphate sodium salt as an effective ingredient.

In the present invention, there is also provided a food or beverage containing an intestinal mineral absorption capacity improver having glucose 1-phosphate sodium salt as an effective ingredient.

In the present invention, there is further provided use of glucose 1-phosphate sodium salt for the preparation of an intestinal mineral absorption capacity improver.

In the present invention, there is further provided a method of improving intestinal mineral absorption capacity, which comprises administering an effective amount of glucose 1-phosphate sodium salt.

### Brief Description of the Drawings

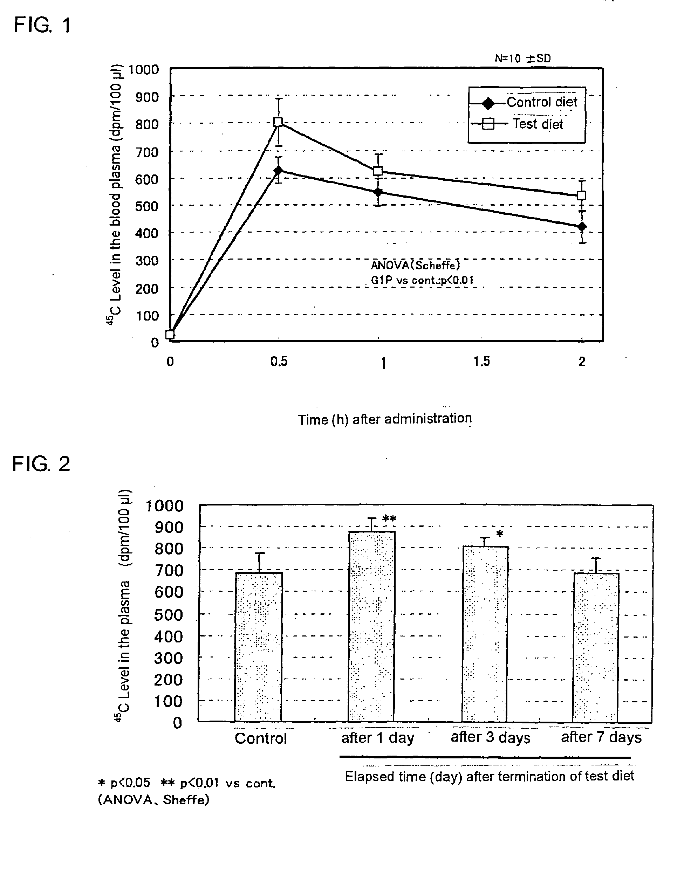

FIG. 1 illustrates a change in the intestinal calcium absorption capacity brought by oral administration of glucose 1-phosphate sodium salt. FIG. 2 illustrates how long the intestinal calcium absorption capacity improving effects last after the administration of glucose 1-phosphate sodium salt is terminated.

### Detailed Description of the Invention

There is a demand for the development of an intestinal mineral absorption capacity improver which, different from conventional mineral supplements or mineral absorption enhancers, does not require intake of it simultaneously with supplementation of minerals. Accordingly, the present invention relates to a highly-safe intestinal mineral absorption capacity improver.

The present inventors have carried out an investigation on the intestinal mineral absorption capacity improving effects of various substances. As a result, it has been found that glucose 1-phosphate sodium salt acts to improve the mineral absorption capacity in the intestinal tract after oral administration; since glucose 1-phosphate sodium salt has considerably higher water solubility and can be orally taken easily compared with calcium glucose-1-phosphate, it can be prepared as an improver without limitation; and its stability higher than that of glucose-1-phosphoric acid facilitates handling of it and preparation of an improver.

The intestinal mineral absorption capacity improver of the present invention has glucose 1-phosphate sodium salt as an effective ingredient. Glucose 1-phosphate sodium salt includes glucose-1-phosphate monosodium and glucose-1-phosphate disodium. Either one of them or a mixture thereof can be used in the present invention. The glucose 1-phosphate sodium salt may be in the form of a hydrate.

Compared with glucose 1-phosphate calcium salt, glucose 1-phosphate sodium salt is higher in water solubility by about 15 times, can be orally taken easily and is superior in long-term intake. It can be designed into a liquid formulation or liquid food. Glucose-1-phosphoric acid is in the form of a starch syrup and has markedly high hygroscopicity so that its formulation designing is difficult. Moreover, it is strongly acidic, for example, a 2 mass% aqueous solution of it has a pH of 1.5, and is therefore unstable, while glucose 1-phosphate sodium salt is in the solid form at normal temperature and a 2 mass% aqueous solution of it has a pH of about 8.0. The glucose 1-phosphate sodium salt is therefore superior in formulation designing and stability.

The glucose 1-phosphate sodium salt can be produced in a large amount at a low cost by the immobilized enzyme method or fermentation method using bacteria as described, for example, in Japanese Patent Application Laid-Open No. Sho 61-22096, Japanese Patent Application Laid-Open No. Hei 1-639589 or Japanese Patent Application Laid-Open No. 2002-300989. The intestinal mineral absorption capacity improver of the present invention can be prepared based on the above-described processes. Glucose 1-phosphate sodium salt may be used after isolation and purification, but a fermentation product of it may be used as is.

As described later in Examples, orally administered glucose 1-phosphate sodium salt activates the mineral absorption capacity of the intestinal tract itself. This reveals that glucose 1-phosphate sodium salt is effective for improving the absorption of minerals which are taken several days later. Simultaneous intake of minerals is not required. The glucose 1-phosphate sodium salt is therefore useful as an improver of the intestinal mineral absorption capacity.

As the dosage form of the intestinal mineral absorption capacity improver of the present invention, any dosage form conventionally used for orally administrable preparations can be employed. Preferred examples include powders, tablets, granules, powders, capsules, aqueous solution and suspension. The intestinal mineral absorption capacity improver of the present invention can also be used as a food, for example, functional food. Its form is not particularly limited. Examples include juices; vegetable juices; seasonings such as sauce, miso and say sauce; soybean products such as soymilk and fermented soybeans; emulsified products such as cream, dressing, mayonnaise and margarine; processed marine products; processed meat products; beverages such as soft drink, liquor, coffee, cocoa, black tea, green tea, fermented tea, semi-fermented tea, powdered beverage and functional beverage; pickles; noodles; soups including powdered soup; jelly; milk products such as cheese, yogurt, and milk; flour products such as bread and cake; confectionery products such as snack, chewing gum, candy and chocolate; and health foods in the form of tablets or granules.

The intestinal mineral absorption capacity improver of the present invention can be used in combination with vitamins such as vitamin D, 1α, 25-(OH)₂ vitamin D, 25-OH vitamin D, tocopherol, ascorbic acid or derivatives thereof, phylloquinone, menaquinone, menadione and derivatives thereof, β-carotene, vitamin A, thiamine, riboflavin, vitamin B6, cyanocobalamin, and folic acid; saccharides such as oligosaccharide, hexose, pentose and sugar alcohol; alcohols such as ethanol; organic acids such as citric acid and malic acid; peptides and proteins such as milk-derived proteins, e.g., whey protein and casein, and soybean protein; flavonoids such as catechin and isoflavone; proteoglycans such as chondroitin sulfate and dermatan sulfate; and extracts and crude drugs such as clove, Juniper Berry, and *Thujopsis dolabrata.* It can also be used in combination with a thickener, gelling agent or emulsion stabilizer such as carrageenan, carboxymethylcellulose, guar gum, pectin, xanthan gum, gum arabic, locust bean gum, tragacanth gum, gellan gum, alginic acid, dextran, pullulan or curdlan.

The intestinal mineral absorption capacity improver of the present invention is preferably administered in an adult dose of from 0.05 to 15 g, especially preferably from 0.1 to 10 g daily, each in terms of glucose 1-phosphate sodium salt. The intestinal mineral absorption capacity improver of the present invention enhances the mineral absorption capacity of the intestinal tract itself as described above so that simultaneous administration of minerals is not necessary, but they may be administered simultaneously or they may be incorporated in the preparation.

The intestinal mineral absorption capacity improver of the present invention can safely activate the mineral absorption capacity of the intestinal tract itself.

### Examples

### Example 1

Rats (8-week-old, male, SD rats), 10 rats consisting of one group, were each fed with 15 g/day of a control diet or a glucose 1-phosphate sodium salt diet as shown in Table 1 for 10 days. As the glucose 1-phosphate sodium salt, product of Solchem (Italy) was employed and the feed was adjusted to have a calcium concentration of 0.2 mass% and a P concentration of 0.4 mass%. The rats were allowed to take distilled water *ad libitum.*

After the rats were fasted for 24 hours, they were orally administered with 1.5 mL (containing ⁴⁵CalµCi), per average weight, of a 1 mg/mL calcium chloride solution containing ⁴⁵Ca singly. The blood plasma was obtained from about 0.5 mL of the blood collected from their carotid artery 30 minutes, 1 hour and 2 hours after administration. The ⁴⁵Ca level in 100 µL of the blood plasma was measured by a liquid scintillation counter.

**Table 1**

| Composition of the feed (mass%) | | |
|---|---|---|
| Composition | Control diet | Test diet |
| Casein | 20.00 | 20.00 |
| DL-Methionine | 0.30 | 0.30 |
| α-Potato starch | 15.00 | 15.00 |
| Sucrose | 47.94 | 46.75 |
| Cellulose powder | 5.00 | 5.00 |
| Corn oil | 5.00 | 5.00 |
| Minerals (Ca free) | 3.50 | 3.50 |
| Ca carbonate | 0.50 | 0.50 |
| Glucose 1-phosphate sodium salt | 0 | 1.88 |
| Potassium dihydrogen phosphate | 1.76 | 1.08 |
| Vitamins (AIN 76) | 1.00 | 1.00 |
| Total | 100 | 100 |

### Results

A change in the ⁴⁵Ca level in 100 µL of the blood plasma separated from the venous blood 30 minutes, 1 hour and 2 hours after the administration of a ⁴⁵Ca-containing calcium chloride solution was statically analyzed by the Scheffe method. As a result, a statistically significant increase in the ⁴⁵Ca level in the blood plasma was observed in the test diet group compared with the control diet group (significant level less than 1%) (FIG. 1). Judging from the fact that after fasting for 24 hours, there remains no glucose 1-phosphate sodium salt in the intestinal tract, particularly in the small intestine, continuous administration of glucose 1-phosphate sodium salt activates the mineral absorption capacity of the intestinal tract itself without the existence of glucose 1-phosphate sodium salt in the intestinal tract upon mineral absorption.

### Example 2

### Method

As in Example 1, 15 g/day of a control diet and a test diet as shown in Table 1 were fed to groups of rats (8-week-old, male, SD rats), each group consisting of 8 rats, for 10 days, respectively. The rat groups fed with the test diet were divided into three groups and as the feed after 10 days, the control diet was fed to each group and feeding of the glucose 1-phosphate sodium salt was terminated. The termination period was 1 day, 3 days and 7 days. The ⁴⁵Ca level in the blood plasma after completion of the feeding period was measured in accordance with the method of Example 1.

### Results

For 3 days after the feeding of glucose 1-phosphate sodium salt was terminated, the ⁴⁵Ca level in the plasma showed a significant increase relative to the control group free of glucose 1-phosphate sodium salt. This suggests that the effect of glucose 1-phosphate sodium salt on mineral absorption enhancement continued for several days and frequent intake of glucose 1-phosphate sodium salt is not necessary.

### Example 3: Food additive

A convenient food additive can be obtained by mixing 60 mass% of glucose 1-phosphate sodium salt with 40 mass% of dextrin and wrapping the mixture in wrappers, each 2 g, or forming the mixture into tablets, each 2 g, in a conventional manner. One wrapper of the mixture or one tablet is suited as an additive used upon cooking for one person.

### Example 4: Powdered beverage

Powdered beverage having good flavor and taste and being taken once a day after dissolved in water or hot water can be obtained by adding 4 mass% of a flavor (lemon flavor) to 60 mass% of glucose, 15 mass% of dextrin, 5 mass% of sodium citrate, 1 mass% of vitamin C and 15 mass% of glucose 1-phosphate sodium salt, mixing them sufficiently, dispensing the mixture by 10 g, and packing each 10 g in a bag.

### Example 5: Tablet candy

A tablet candy which has good flavor and taste and exerts its effect by intake of 5 tablets a day can be prepared by mixing 60 mass% of anhydrous crystalline glucose, 9 mass% of frost sugar, 4.5 wt.% of powdered orange flavor, 2 mass% of guar gum, 2.5 mass% of ascorbic acid, 1.5 mass% of citric acid (crystal), 5 mass% of milk calcium, 15 mass% of glucose 1-phosphate sodium salt, 0.5 mass% of sucrose fatty acid ester (HLB20) and an adequate amount of a colorant; and tableting the mixture into 15 mmφ tablets, each 2 g, in a conventional manner.

### Example 6: Jelly

A jelly which melts well in the mouth, has a fruit flavor, provides good palatability and has good flavor and taste can be obtained by mixing a 0.65 mass% of a gelling agent mixture of carrageenan and locust been gum, 10 mass% of a 50% concentrated grape fruit juice, 0.05 mass% of citric acid, 0.05 mass% of vitamin C, and 1.5 mass% of glucose 1-phosphate sodium salt with 40 mass% of water, adding an adequate amount of a grape fruit flavor to the resulting mixture, adding an adequate amount of a liquid sugar, adding water to the mixture to obtain 1 kg of a 100 mass% of the solution, sterilizing the solution by keeping it at 85°C for 5 minutes, dispensing it into a 100 mL container, allowing it to stand and gradually cooling it to 5°C for cause gelation.

### Example 7: Milk drink

A milk drink can be obtained by adding 30 g of glucose 1-phosphate sodium salt in 5 kg of commercially available milk to dissolve the former in the latter, packing the mixture in a can and sterilizing it in a conventional manner.

### Example 8: Soft drink

Water is added to 13 mass% of a high fructose corn syrup, 0.3 mass% of citric acid, 0.03 mass% of ascorbic acid, 0.02 mass% of sodium citrate, 0.1 mass% of a flavor (lime flavor), 0.5 mass% of calcium carbonate and 1.2 mass% of glucose 1-phosphate sodium salt to dissolve the latter in the former and the resulting solution is adjusted to pH 3.5, whereby 5 liter of a solution is obtained. A soft drink with good flavor and taste can be obtained by dispensing the solution in a 100 mL glass bottle, and then sterilizing it in a conventional manner.

### Example 9: Water

Bottled water can be obtained by dissolving 30 g of glucose 1-phosphate sodium salt in 5 kg of commercially available mineral water, filtering the resulting solution, filling a 200 mL can bottle therewith and then sterilizing it in a conventional manner.

### Example 10: Tea drink

Oolong tea with good flavor and taste can be obtained by preparing 5 kg of a tea extract from 50 g of Oolong tea leaves, adding 20 g of glucose 1-phosphate sodium salt and 1 g of sodium ascorbate to the extract to dissolve the former in the latter, adjusting the pH of the resulting solution to 5.5 with sodium bicarbonate, packing the mixture in a 350 mL can, and sterilizing it in a conventional manner.

### Example 11: Coffee drink

A coffee drink with good flavor and taste can be obtained by preparing 4 kg of a coffee extract from 250 g of roasted coffee beans (Mocha and Columbia), adding 200 g of sucrose and 500 g of milk to the extract, adding 30 g of glucose 1-phosphate sodium salt to the mixture to dissolve the former in the latter, adjusting the total quantity and pH of the solution to 5 kg and pH 6 with water and sodium bicarbonate, respectively, packing 200 mL of the solution in a can and sterilizing it in a conventional manner.

### Example 12: Vegetable-containing Mix juice

A vegetable-containing mix juice can be obtained by adding 12 g of glucose 1-phosphate sodium salt to 2 kg of a commercially available mix juice to dissolve the former in the latter, packing 200 mL of the resulting solution in a can, and sterilizing it in a conventional manner.

### Example 13: Ice cream

Ice cream with good flavor and taste can be obtained by adding 60 g of sucrose, 30 g of glucose 1-phosphate sodium salt and an adequate amount of flavor to 2 kg of milk, stirring the resulting mixture while cooling until smooth and creamy, dividing the cream into 20 portions, and then cooling them further.

### Example 14: Flour product (cupcake)

A cupcake with good flavor and taste can be obtained by making a dough from 100 g of all-purpose flour, 100 g of a hen egg, 100 g of sugar, 35 g of shortening, 35 g of margarine, 2.5 g of baking powder, 6 g of a liquor, 15 g of glucose 1-phosphate sodium salt and an adequate amount of water in a manner conventionally employed for a cupcake, dividing the resulting dough and putting them into 10 baking cups, and baking them in a conventional manner.

### Example 15: Seasoning (say sauce)

Say sauce or low-salt say sauce can be obtained by adding 100 g of glucose 1-phosphate sodium salt to commercially available say sauce or low-salt say sauce to dissolve the former in the latter, sterilizing the solution, and adjusting a daily using amount to 20 g.

### Example 16: Health drink

A health drink with good flavor and taste can be obtained by adding 800 mg of taurine, 11000 mg of sucrose, 50 mg of caramel, 30 mg of sodium benzoate, 5 mg of vitamin B1 nitrate, 20 mg of vitamin B2, 20 mg of vitamin B6, 2000 mg of vitamin C, 100 mg of vitamin E, 20 mg of nicotinic amide, and 1200 mg of glucose 1-phosphate sodium salt to an adequate amount of purified water to dissolve the former in the latter, adjusting the resulting solution to pH 3 with an aqueous phosphoric acid solution, adding purified water to give a total amount of 100 mL, and sterilizing the solution at 80°C for 30 minutes.

## Claims

1. An intestinal mineral absorption capacity improver comprising glucose 1-phosphate sodium salt as an effective ingredient.

2. The intestinal mineral absorption capacity improver of Claim 1, which is for oral administration.

3. The intestinal mineral absorption capacity improver of Claim 1, which is in the form of an orally administrable liquid.

4. A food or beverage comprising the intestinal mineral absorption capacity improver as claimed in any one of Claims 1 to 3.

5. Use of glucose 1-phosphate sodium salt for the preparation of an intestinal mineral absorption capacity improver.

6. The use of Claim 5, wherein the intestinal mineral absorption capacity improver is for oral administration.

7. The use of Claim 5, wherein the intestinal mineral absorption capacity improver is in the form of an orally administrable liquid.

8. Use of glucose 1-phosphate sodium salt for the preparation of a food or beverage for improving intestinal mineral absorption capacity.
